# EUROPEAN PATENT SPECIFICATION

(11) **EP 3 938 764 B1**
(45) Date of publication and mention of the grant of the patent: **25.11.2026**
(21) Application number: 20801854.9
(22) Date of filing: 04.05.2020
(51) Int. Cl.: G01N 21/77, G01N 1/28, G01N 21/82, G01N 33/53, G01N 1/40

(54) **DIGITAL MICROFLUIDIC AGGLUTINATION ASSAYS**
DIGITALE MIKROFLUIDISCHE AGGLUTINATIONSTESTS
DOSAGES NUMÉRIQUES D'AGGLUTINATION MICROFLUIDIQUE

(30) Priority: 03.05.2019 GB 201906331
(43) Date of publication of application: 19.01.2022
(73) Proprietor: The Governing Council of the University of Toronto, Toronto, Ontario M5G 1L7 (CA)
(72) Inventor: SKLAVOUNOS, Alexandros, Toronto, Ontario M5S 3A6 (CA); LAMANNA, Julian Lucas, Toronto, Ontario M4C 2S7 (CA); WHEELER, Aaron R., Toronto, Ontario M6H 3M4 (CA)
(74) Representative: FRKelly
(86) International application number: PCT/CA2020/050592
(87) International publication number: WO 2020/223801

(56) References cited:
- WO-A2-2004/027028
- US-A1- 2002 031 791
- US-A1- 2011 104 725
- US-A1- 2018 126 381
- US-A1- 2018 126 381
- US-A1- 2018 238 871
- US-A1- 2018 238 871
- US-A1- 2018 275 088
- US-B2- 6 878 558
- MARCAH MERVE: "DEVELOPMENT OF DROPLET BASED MICROFLUIDIC SYSTEM FOR AGGLUTINATION ASSAYS", 1 October 2015 (2015-10-01), XP055970929, Retrieved from the Internet <URL:https://acikbilim.yok.gov.tr/bitstream/handle/20.500.12812/34371/yokAcikBilim_10091423.pdf?sequence=-1&isAllowed=y> [retrieved on 20221013]

## Description

### FIELD

The present disclosure relates to a method for performing agglutination assays using a "two plate" digital microfluidic (DMF) device format. Droplets containing analytes of interest (particles, cells, etc.) are loaded into the DMF device and mixed with solution-phase or dried agglutinating antibodies or antigens.

### BACKGROUND

Agglutination assays are commonly used for the detection of the presence of analytes in a sample; typical applications include infectious disease and pathogen detection, and blood typing for donor compatibility. Agglutination assays rely on antibody or antigen interactions with an analyte of interest; the result of this interaction is the formation of large, insoluble clumps or aggregates that are visible to the eye. Thus, agglutination assays have a unique advantage relative to other techniques that are used for such assays (which rely on instrumental measurement of photonic or electrical energy) - the results of the assay are very straightforward to read.

In traditional agglutination assays, particles coated with antibodies or antigens are combined with a sample, manually mixed, and the presence of aggregates is determined by visual inspection. Obvious drawbacks of the standard technique include the requirement for manual mixing, the potential for errors in interpretation of the assay's readout, and low throughput. Agglutination assays in microfluidic devices have been recently developed in efforts to address these drawbacks. For example, Castro et al.¹ described a microfluidic method for an agglutination assay that relies on hydrodynamic forces for mixing combined with imaging-based detection, which was reported to be useful for limiting user input and minimizing analytical variability. Other microfluidic implementations of agglutination assays have relied on flow cytometry,² light scattering,³ fluorescence,⁴ and optical microscopy.^{5,6} These detection methods are useful for method development and optimization, but they require ancillary detectors that (ultimately) negate the primary advantage of agglutination - the ability to read the results of the test without the need for complex detection schemes.

Another challenge affecting previously reported microfluidic based agglutination assays is the requirement that sample must be diluted prior to introduction to the microfluidic device, increasing the complexity of the assay.⁵ An ideal method would accept un-processed sample, which would be compatible with use by non-experts. In addition, manipulation of the agglutinates in narrow channels can cause channel clogging, which can lead to device failure and problems with reliability. Finally, as an alternative to conventional microfluidics, there are many examples of agglutination assay methods implemented in lateral flow-based "paper microfluidics" format (e.g., Yoon and You⁷), but these methods typically have low throughput and/or require manual wash steps. Here, we introduce a new method relying on digital microfluidics (DMF) that overcomes the limitations of the techniques described above.

DMF is a liquid handling technology that uses electrostatic forces to manipulate picoliter to microliter sized droplets of liquid. The most powerful format of DMF is the "two plate" configuration in which droplets are sandwiched between a counter electrode top plate and bottom plate baring an array of insulated electrodes. When operated in two-plate format, droplets can be dispensed, split, merged, and mixed, making DMF extremely useful for sample processing,⁸ immunoassays,⁹⁻¹¹ and chemical reactions.¹² DMF has numerous differences relative to traditional microfluidics, including versatility (a generic device architecture can be applied to a variety of applications), absolute control over the position of reagents (liquid or dried) without requiring moving parts, and operation in an open geometry, with no chance for channel clogging.

The present inventors are aware of two previous reports of agglutination assays implemented on DMF devices. One method used back-light scattering detection for a latex immunoagglutination assay,⁷ and another used functionalized gold nanoparticles to agglutinate a biomarker of interest, coupled with a micro separation procedure for detection.¹³ Note that neither of these techniques was demonstrated in the "standard" format for digital microfluidics That is, the first⁷ does not use electric fields to manipulate droplets (in contrast, an XYZ-stage-controlled wire is used to mechanically push/pull droplets around the surface), and the second¹³ uses so-called "single plate" DMF (in contrast to the more powerful "two plate" DMF format used in the method disclosed herein). These "non-standard" DMF formats^{7,13} were useful for proof-of-concept, but we propose that they are almost certainly not compatible with the type of fully integrated sample-in-answer-out system that has become common-plate for two-plate digital microfluidics.¹¹

The term digital microfluidics (DMF) has been broadly used to describe liquid droplet manipulation systems. Several fluidic actuators have been reported such as using chemical¹⁴ or thermal gradient,¹⁵ magnets,¹⁶ acoustic waves,¹⁷ mechanical⁷ and electrical methods.¹³ Since the present invention relies on the use of electrostatic liquid manipulation forces, often referred to as electrowetting on dielectric (EWOD), we will focus only on the comparison between one-plate and two-plate DMF EWOD devices, all other devices (non EWOD) are not going to be discussed further as they use other liquid manipulation techniques irrelevant to the present invention.

DMF EWOD systems are often divided into two main categories; single plate DMF and two-plate DMF. There are several reasons and significant technical challenges to move from a single plate DMF device to a two-plate DMF device. The one-plate DMF device term is used to describe open systems where droplets are sitting freely on a horizontal solid substrate and while the term two-plate DMF device is used to describe covered systems where the droplet is confined between two plates. Both types of devices require sufficient grounding for operation; one-plate devices require either an external wire that comes in direct contact with the droplet or an electrode within the same plane as the actuating electrode while in two-plate devices the ground is located within the top plate.

Beyond the number of plates, these two types of DMF devices are quite different in terms of their abilities to perform droplet operations. Droplet motion is easier in two-plate systems. Additionally, splitting and dispensing of droplets is almost an exclusive option of two-plate systems. On the contrary, one-plate devices are preferred when vigorous mixing, evaporation (for species concentration) and direct access to the liquid droplet is required, since the droplet is readily available. One-plate devices often operate at much higher voltages and lower frequencies, which requires a different hardware instrumentation than the two-plate systems. Therefore, despite the fact that there have been previous reports of digital microfluidics used for agglutination assays,^{7,13} it is not obvious how one could transition an agglutination from these devices to a two-plate DMF system as there are numerous technical differences between the two types of devices which needed to be optimized and determined in order to develop agglutination assays on the two-plate DMF device reported here.

Furthermore, even from the perspective of agglutination assays, neither of the previous reports is ideal -the back-scatter technique⁷ requires ancillary instrumentation for analysis, and the nanoparticle-based technique¹³ is slow (as the user must wait for each sample to evaporate prior to analysis) and requires a custom, expensive, nano-particle based reagent. We note that both methods were reported more than ten years ago, with no follow-up publications, suggesting slow (or no) uptake by the community.

US2018/275088A1 discloses systems and methods for analyte analysis using a nanopore.

US2018/126381A1 relates to an instrument for the detection of an analyte in a sample.

US2011/104725A1 discloses a method for coagulating blood on a droplet actuator.

Merve Marcah: "DEVELOPMENT OF DROPLET BASED MICROFLUIDIC SYSTEM FOR AGGLUTINATION ASSAYS", 1 October 2015, XP055970929, relates to a method of detecting agglutination reactions of whole blood in micro droplets using impedimetric measurements.

US2018/238871A1 relates to a reagent and method for assaying the thrombin-antithrombin complex (TAT) in a blood sample.

US6878558B2 discloses a whole blood immunoassay including the step of mixing a whole blood sample with sensitized insoluble carrier particles to cause agglutination. The degree of agglutination is determined to be the ratio of the number of agglutinated particles to the total number of particles in the sample (agglutinated and unagglutinated).

### SUMMARY

The present disclosure provides a novel method for performing agglutination assays on a "two plate" digital microfluidic (DMF) device format. Droplets containing analytes of interest (particles, cells, etc.) are loaded into the DMF device and mixed with solution-phase or dried agglutinating antibodies or antigens. The agglutinating agents bind to their complementary targets (e.g. antibodies or antigens) in the sample droplets, which leads to the formation of insoluble aggregates. Active mixing on DMF reduces the reaction time and enhances the agglutination effect. Since the agglutinated sample is sandwiched between two plates on the DMF device, it is straightforward to visualize the result by eye or via a digital camera.

Thus the present disclosure provides a method of characterizing a sample containing analytes using a two-plate electrowetting digital microfluidic device (DMF) having a plurality of driving electrodes, the method comprising steps of:
providing a two-plate electrowetting digital microfluidic device (DMF) having a plurality of driving electrodes; loading a fluid sample containing the analytes and an agglutination agent capable of causing agglutination onto separate driving electrodes of said DMF device; using electrowetting for bringing droplets of the fluid sample in contact with the agglutination agent for agglutination of any of the analytes present in the sample with the agglutination agent to produce an agglutinate; and visually characterizing any agglutinate formed due to the presence of the pre-selected analytes in the fluid sample by: using a camera positioned at an angle to collect an image of the DMF device; performing perspective correction; locating the center of the DMF device automatically by detecting known device features, and isolating the region of the image for further processing; detecting the droplets by identifying contours and combining neighbouring contours to form a rectangular region of interest for each droplet; masking and isolating the region of interest image corresponding to each droplet, and converting for each droplet the region of interest image into gray scale; flattening each isolated image into a one-dimensional array which is normalized such that pixel intensities cover a full 8-bit range, and sorted by pixel-value from lowest to highest; using a slope of pixel intensity as an indication of a degree of agglutination; and determining the degree of agglutination for each of the regions of interest based on a slope of a gradient of the sorted pixel-intensity array.

The method may further comprise a step of characterizing an amount of agglutination of the analytes caused by the agglutination agent.

The fluids loaded on said DMF device may contain a surfactant.

The surfactant may be in a pre-dried form, and the method may further comprise coating one or more driving electrodes with the pre-dried form of the surfactant, either in pre-determined spots or coated across the entire device surface, such that when the fluid sample comes into contact with the pre-dried surfactant, it becomes solubilized, and the surfactant may be present in an amount of at least 0.01% wt:wt in the fluid. The process of drying and reconstituting reagents on the DMF device may be performed in accordance with the methods described in US 2014/0141409 A1 (Foley et al.¹⁸).

The surfactant may be one of an ionic surfactant and a non-ionic surfactant. The ionic surfactants may be selected from the group consisting of sodium dodecyl sulfate, sodium stearate, cetrimonium bromide, cetrimonium chloride, and sodium lauryl sulfate. Non-ionic surfactants include but are not limited to alkylphenol hydroxypolyethylenes (e.g. Triton X RTM), polysorbates (e.g. Tween RTM), poloxamines (e.g. Tetronic RTM), poloxamers (e.g. Pluronic RTM), and sorbitan esters. The poloxamers may comprise Pluronic^{®}.

The agglutination agent may be a liquid loaded and metered to preselected driving electrodes.

The agglutination agent may be in a pre-dried form, and the method may further comprise coating one or more driving electrodes with the pre-dried form of the agglutination agent in pre-determined spots of the surface of said DMF device, such that when a fluid comes into contact with the pre-dried agglutination agent, it becomes solubilized. The process of drying and reconstituting reagents on the DMF device may be performed in accordance with the methods described in US 2014/0141409 A1 (Foley et al.¹⁸).

The method may further comprise a step of actively mixing the agglutination agent with the fluid sample using electrowetting on the DMF device.

The agglutination agent may comprise any one or combination of substances capable of producing an agglutinate. Examples of substances include chemical agglutination agents and biological agglutination agents. For agglutination of red blood cells a chemical agglutination agent may be selected from the group consisting of poly-L-lysine hydrobromide, poly(dimethyl diallyl ammonium) chloride (Merquat^{®}-100 RTM, Merquat^{®}-280 RTM, Merquat^{®}-550 RTM), poly-L-arginine hydrochloride, poly-L-histidine, poly(4-vinylpyridine), poly(4-vinylpyridine) hydrochloride, poly(4-vinylpyridine)crosslinked, methyl chloride quaternary salt, poly(4-vinylpyridine-co-styrene); poly(4-vinylpyridinium poly(hydrogen fluoride)); poly(4-vinylpyridinium-P-toluenesulfonate); poly(4-vinylpyridinium-tribromide); poly(4-vinylpyrrolidone-co-2-dimethylaminoethyl methacrylate); poly vinylpyrrolidone, cross-linked; poly vinylpyrrolidone, poly(melamine-co-formaldehyde); partially methylated; hexadimethrine bromide; poly(Glu, Lys) 1:4 hydrobromide; poly(Lys, Ala) 3:1 hydrobromide; poly(Lys, Ala) 2:1 hydro-bromide; poly-L-lysine succinylated; poly(Lys, Ala) 1:1 hydrobromide; and poly(Lys, Trp) 1:4 hydrobromide.

The chemical agglutination agent may be poly (dimethyl diallyl ammonium) chloride.

The biological agglutination agent may be selected from the group consisting of proteins, antibodies, viruses and antigens, DNA, RNA and DNA or RNA based aptamers.

The proteins may comprise lectins able to reversibly bind saccharide structures. The antibodies may comprise Anti-A, Anti-B and Anti-D.

The viruses may comprise influenza virus.

The step of characterizing an amount of agglutination of the analytes caused by the agglutination agent includes visual characterization. This visual characterization may, in an example not falling under the scope of the invention, be by a person observing the DMF device to approximate the amount of agglutination. According to the invention, the step of visual characterization is performed using a camera. The camera may be any one of webcams, cell phone cameras, digital camera (including digital single-lens reflex camera, DSLR), video cameras, surveillance cameras point and shoot cameras, cameras with CCD detectors, cameras with CMOS detectors, monochrome cameras, black and white cameras, color cameras.

Particles may be coated with the agglutination agent. These may include any one or combination of polymer particles (e.g. latex), gold, silver, nano- and micro- particles. The polymer particles may comprise latex.

The analytes being detected for may be antibodies, and wherein the particles may be coated with an antigen or other agent capable of capturing the antibody of interest.

The analytes being detected for may be antigens and wherein the particles may be coated with an antibody or other agent capable of capturing the antigen of interest.

The analytes being detected for may be bacteria and wherein the particles may be coated with an antibody or other agent capable of capturing the bacterium of interests.

The analytes being detected for may be viruses and wherein the particles may be coated with an antibody or other agent capable of capturing the virus of interests.

The method may be used for agglutination of a suspension of polymer particles.

The method may be used for agglutination of a suspension of nanoparticles.

The method may be used for agglutination of a suspension of red blood cells.

The fluid may be a blood sample comprising at least red blood cells.

The fluid may contain a virus suspension for detection of viruses using agglutination of red blood cells or particles.

The method may be used for agglutination of a suspension of white blood cells.

The fluid may be a serum sample or a plasma sample comprising at least white blood cells.

The method may be used for agglutination of a suspension of eukaryotic cells of any other type.

The agglutination agent may be any substance capable of causing agglutination of cells.

Cells may be red blood cells.

The agglutination agent when used with red blood cells may be used for the determination of hematocrit level.

The present provides a two-plate electrowetting DMF device, comprising:
a first plate, a second plate spaced from the first plate, one of the first and second plates having a plurality of driving electrodes; and
a surface on either the first plate or the second plate having a surfactant in a pre-dried form coating the surface in preselected locations or coating the entire plate, another surface on either the first plate or the second plate having an agglutination agent in a pre-dried form coating the other surface in preselected locations; a camera; and means for image processing configured to perform the imaging processing as defined in claim 1.

The surface coated by the surfactant and the surface coated with the agglutination agent are either different or the same.

The present disclosure provides a kit, comprising:
a two-plate electrowetting digital microfluidic device (DMF) having a plurality of driving electrodes;
a surfactant for placement on one of the two plates; and
an agglutination agent for placement on one of the two plates.

The agglutination agent may be selected for agglutination of red blood cells.

A further understanding of the functional and advantageous aspects of the disclosure can be realized by reference to the following detailed description and drawings.

### BRIEF DESCRIPTION OF THE DRAWINGS

Embodiments will now be described, by way of example only, with reference to the drawings, in which:

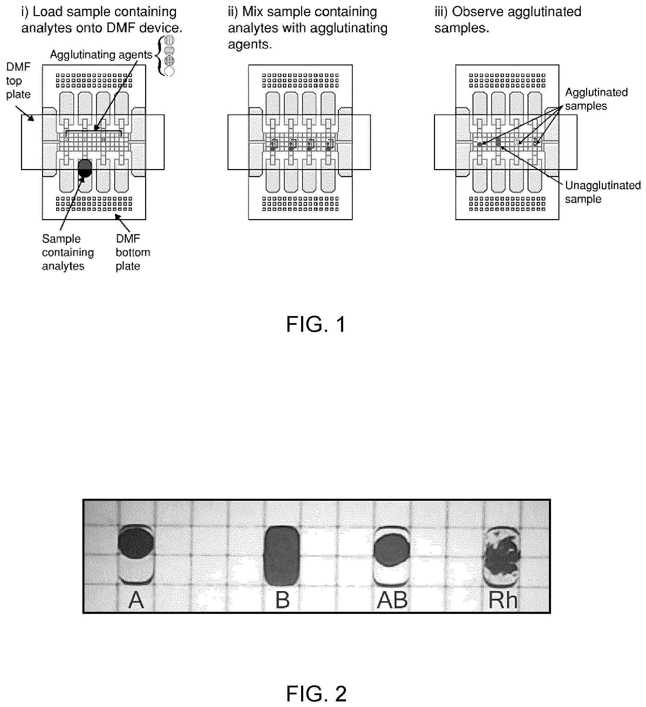

**FIG. 1** shows in three (3) panels a DMF device and the associated steps of an agglutination assay on a DMF device with the left most panel labelled (i) showing one or more samples containing analytes of interest is loaded onto the DMF device which contains agglutinating agents, the middle panel labelled (ii) showing the sample being metered into sub-samples and then each sub-sample is mixed with an agglutinating agent for a pre-determined period of time and the right most panel labelled (iii) show that the agglutination is observed on the DMF device visually or by camera.
**FIG. 2** is a drawing depicting the result of a DMF agglutination assay for blood typing. A whole blood sample was loaded onto the device, after which it was metered into four sub-droplets. Each sub-droplet was mixed with a separate droplet containing anti-A (left), anti-B (second from left), anti-A / anti-B blend (second from right) and anti-D (right) antibodies on a DMF device. After mixing for 2 min, the result can be determined by eye. The particular sample here formed agglutinates with anti-A, anti-AB, and anti-D (Rh), indicating A+ type.

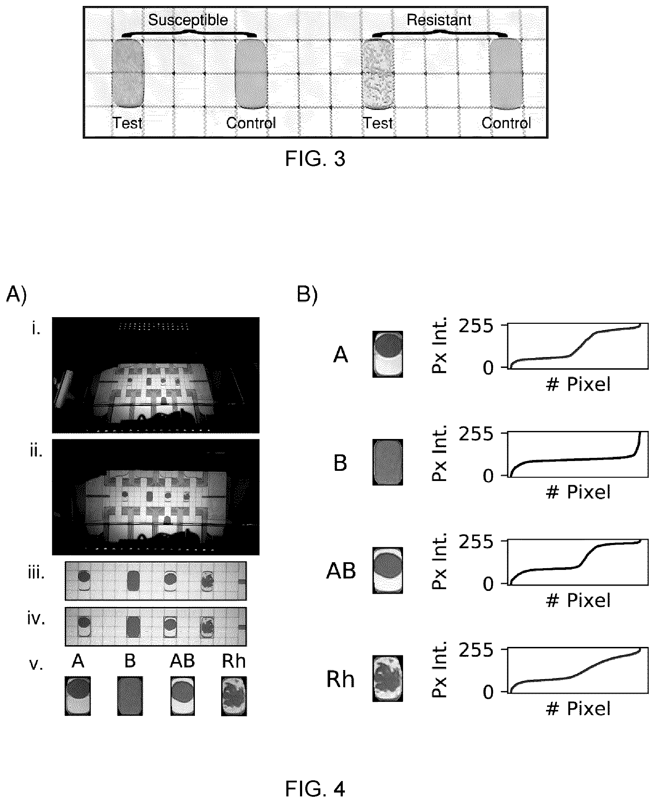

**FIG. 3** is a drawing depicting the result of a bead-based DMF agglutination assay. A first sample of bacteria lysate was loaded into the device and then metered into a pair of sub-droplets that were mixed with a droplet containing latex beads coated with PBP2 antibodies (left), or latex beads coated with antibodies non-specific to PBP2 (second from left). The first sample formed weak agglutinates with the latex beads coated with PBP2 antibodies and no agglutinates with the latex beads coated with antibodies not specific to PBP2, indicating that the bacteria is susceptible to methicillin. Similarly, a second sample of bacteria lysate was loaded into the device and then metered into a pair of sub-droplets that were mixed with a droplet containing latex beads coated with PBP2 antibodies (second from right), and latex beads coated with antibodies that are not specific to PBP2 (right). The second sample formed strong agglutinates with the latex beads coated with PBP2 antibodies and no agglutinates with the latex beads coated with antibodies non-specific to PBP2, indicating that the bacteria is resistant to methicillin. In both cases, after loading the samples, the process was automated, leading to results after ~2 minutes of mixing.
**FIG. 4** is a drawing showing the workflow of automated image analysis for determining the output of DMF agglutination assays. A) Images collected from a digital camera illustrating the steps from initial image capture to isolation of the ROIs (regions of interest). (i) An image of the device is captured at an angle to reduce reflection. (ii) A perspective correction is performed. (iii) The portion of the image featuring the center of the device is isolated. (iv) In the isolated image, the regions of interest (ROIs) are identified for each droplet and (v) each ROI is masked and stored as a separate image for analysis. B) Images (left) and data (right) illustrating the analysis of each ROI. The variation in pixel intensity of each ROI indicates the degree of agglutination.

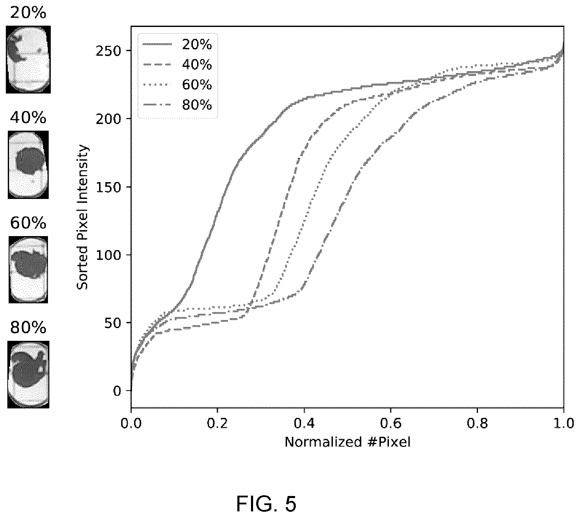

**FIG. 5** is a drawing depicting the result of a DMF agglutination assay for the determination of hematocrit level. In the left four droplets are shown with different hematocrit levels (ratio of the volume of red blood cells to the total volume of blood) - 20% (top), 40 % (second from top), 60% (second from bottom), 80% (bottom). The droplets were mixed with a chemical agglutination agent that causes non-specific agglutination of red blood cells. The higher the hematocrit level the bigger the agglutinated spot will be. The hematocrit level can be estimated by naked eye or determined using a digital camera. On the right, is shown the output of a processed image captured with a digital camera. The difference in the intensity of the pixels can be used to determine the hematocrit level of the sample.

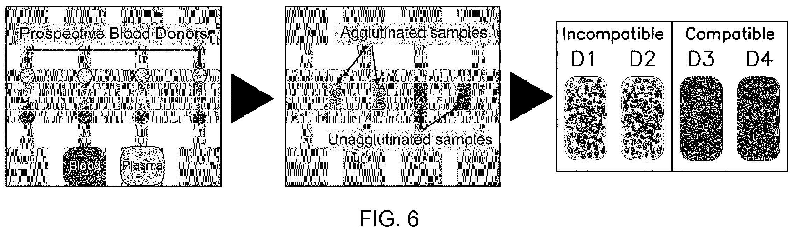

**FIG. 6** is a drawing with three panels depicting the result of a DMF agglutination assay for donor compatibility testing. A whole blood sample was loaded onto the device, after which it was metered into four sub-droplets, as shown in the left most panel. Each sub-droplet was then mixed with a separate droplet containing plasma from prospective blood donors on a DMF device as shown in the middle panel. After mixing for 5 min, the result can be determined by eye. The particular sample here formed agglutinates with the first two samples from the left, D1, D2 (in the right hand panel) indicating that these donor samples are incompatible with the recipient's blood, and the other two samples D3, D4 (on the right) did not show any signs of agglutination indicating that these donor samples are compatible with the recipient's blood.

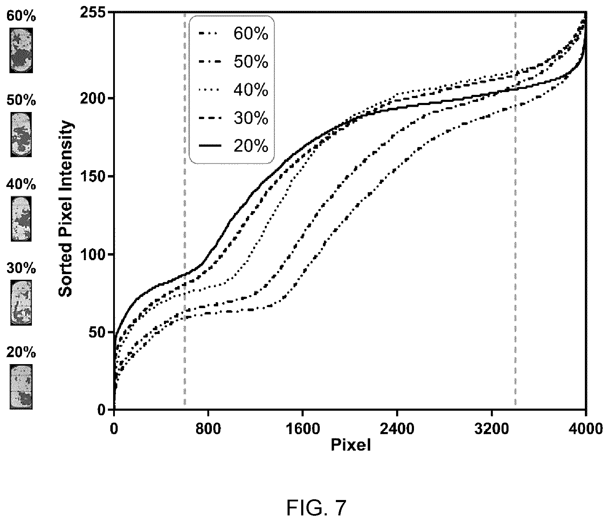

**FIG. 7** is a plot of sorted pixel intensity versus number of pixels depicting the result of a DMF agglutination assay for the determination of hematocrit level. To the left of the vertical axis five droplets are shown with different, artificially defined hematocrit levels between 20 and 60% - 60% (top), 50 % (second from top), 40% (third from top), 30% (second from bottom), 20% (bottom). The droplets were mixed with a chemical agglutination agent that causes non-specific agglutination of red blood cells. The higher the hematocrit level the bigger the agglutinated spot will be. The hematocrit level was determined using image analysis. On the right, is shown the output of a processed image captured with a digital camera. The integral of intensity of the pixels for each sample was used to determine the hematocrit level of the sample.

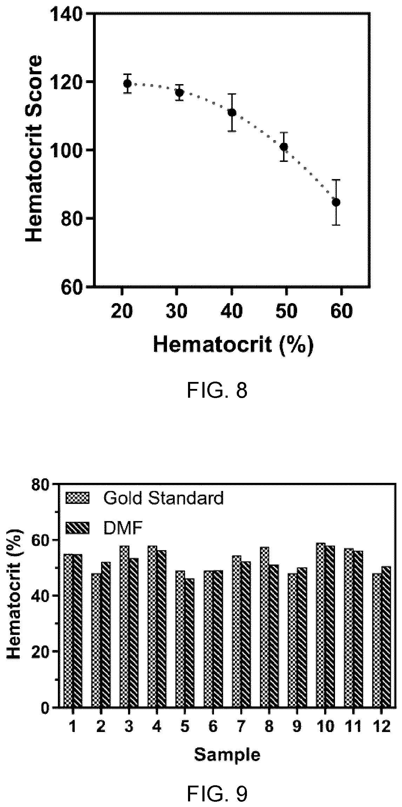

**FIG. 8** is a plot of hematocrit score versus hematocrit (%) depicting the calibration curve generated from the images shown in **FIG. 7****.** The markers are experimental data, and error bars represent ±1 standard deviation for n=3 experiments per condition. The dashed second order polynomial was fitted to the data, with R² = 0.9990.
**FIG. 9** shows a bar graph of hematocrit measurements collected from 12 finger-prick whole blood samples from volunteers using the gold standard (left) and the DMF- droplet agglutination assessment on DMF (DAAD) method (right). For the set of 12 samples comparison between gold standard and DMF-DAAD methods yields p ≥ 0.5045.

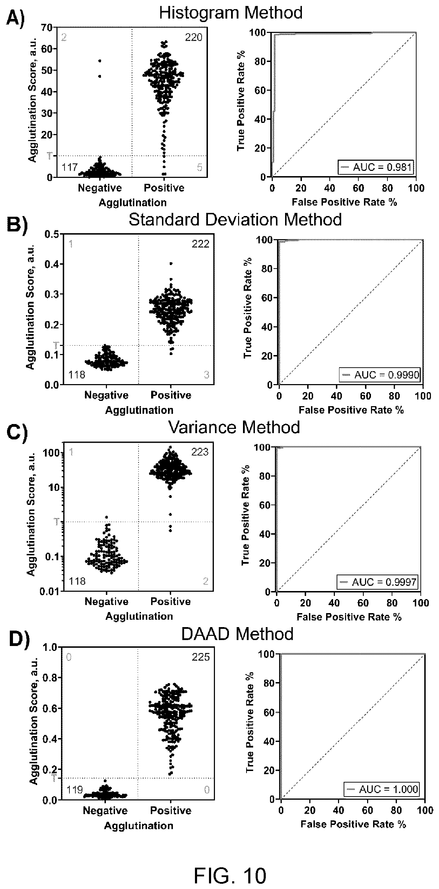

**FIGS. 10A** to **10D** are a series of plots depicting the performance comparison between agglutination detection algorithms for detecting agglutination in 344 sample images. The data were known to include 225 positive and 119 negative samples as defined by the gold standard method. Negative is a sample that did not exhibit any signs of agglutination and Positive is a sample that showed any signs of agglutination.
**FIG. 10A** shows the performance of the histogram method.
**FIG. 10A** left hand panel is a plot of the agglutination scores of the sample images produced by the histogram method versus the gold standard result. Inside the plot, black and gray denote the numbers of correct/incorrect assessments using the threshold T (10 a.u.).
**FIG. 10A** right hand panel is a plot of receiver operating characteristic (ROC) curve showing the method's true positive rate versus the method's false positive rate. The dashed lines in the ROC curves represent the result of random guesses (coin flip). The area under the curve (AUC) of the method is 0.981.
**FIG. 10B** shows the performance of the standard deviation method.
**FIG. 10B** left hand panel is a plot of the agglutination scores of the sample images produced by the standard deviation method versus the gold standard result. Inside the plot, black and gray denote the numbers of correct/incorrect assessments using the threshold T (0.13 a.u.).
**FIG. 10B** right hand panel is a plot of receiver operating characteristic (ROC) curve showing the method's true positive rate versus the method's false positive rate. The dashed lines in the ROC curves represent the result of random guesses (coin flip). The area under the curve (AUC) of the method is 0.9990.
**FIG. 10C** shows the performance of the variance method.
**FIG. 10C** left hand panel is a plot of the agglutination scores of the sample images produced by the variance method versus the gold standard result. Inside the plot, black and gray denote the numbers of correct/incorrect assessments using the threshold T (1 a.u.).
**FIG. 10C** right hand panel is a plot of receiver operating characteristic (ROC) curve showing the method's true positive rate versus the method's false positive rate. The dashed lines in the ROC curves represent the result of random guesses (coin flip). The area under the curve (AUC) of the method is 0.9997.
**FIG. 10D** shows the performance of the droplet agglutination assessment on DMF (DAAD) method.
**FIG. 10D** left hand panel is a plot of the agglutination scores of the sample images produced by the DAAD method versus the gold standard result. Inside the plot, black and gray denote the numbers of correct/incorrect assessments using the threshold T (0.152 a.u.).
**FIG. 10D** right hand panel is a plot of receiver operating characteristic (ROC) curve showing the method's true positive rate versus the method's false positive rate. The dashed lines in the ROC curves represent the result of random guesses (coin flip). The area under the curve (AUC) of the method is 1.000.

### DETAILED DESCRIPTION

Various embodiments and aspects of the disclosure will be described with reference to details discussed below. The following description and drawings are illustrative of the disclosure and are not to be construed as limiting the disclosure. Numerous specific details are described to provide a thorough understanding of various embodiments of the present disclosure. However, in certain instances, well-known or conventional details are not described in order to provide a concise discussion of embodiments of the present disclosure.

As used herein, the terms, "comprises" and "comprising" are to be construed as being inclusive and open ended, and not exclusive. Specifically, when used in the specification and claims, the terms, "comprises" and "comprising" and variations thereof mean the specified features, steps or components are included. These terms are not to be interpreted to exclude the presence of other features, steps or components.

As used herein, the term "exemplary" means "serving as an example, instance, or illustration," and should not be construed as preferred or advantageous over other configurations disclosed herein.

As used herein, the terms "about" and "approximately", when used in conjunction with ranges of dimensions of particles, compositions of mixtures or other physical properties or characteristics, are meant to cover slight variations that may exist in the upper and lower limits of the ranges of dimensions so as to not exclude embodiments where on average most of the dimensions are satisfied but where statistically dimensions may exist outside this region. It is not the intention to exclude embodiments such as these from the present disclosure. Unless otherwise specified, the terms "about" and "approximately" mean plus or minus 25 percent or less.

It is to be understood that unless otherwise specified, any specified range or group is as a shorthand way of referring to each and every member of a range or group individually, as well as each and every possible sub-range or sub-group encompassed therein and similarly with respect to any sub-ranges or sub-groups therein. Unless otherwise specified, the present disclosure relates to and explicitly incorporates each and every specific member and combination of sub-ranges or sub-groups.

As used herein, the term "on the order of", when used in conjunction with a quantity or parameter, refers to a range spanning approximately one tenth to ten times the stated quantity or parameter.

As used herein, "agglutination" refers to a process in which clumps of cells or inert particles are formed due to the interaction between specific antibodies and antigenic components, or due to other chemicals that can induce the same clumping effect.

Agglutination is defined as the formation of clumps of cells or inert particles by specific antibodies to surface antigenic components (direct agglutination) or to antigenic components adsorbed or chemically coupled to red cells or inert particles (passive hemagglutination and passive agglutination, respectively).¹⁹ Erythrocytes are also agglutinated by non-antibody substances such as plant proteins, viruses, salts of heavy metals, inorganic colloidal acids and bases, and basic proteins (protamines, histones). Agglutination inhibition or hemagglutination inhibition refers to the inhibition of these reactions by soluble antigen which reacts with the combining sites of the antibodies and thereby prevents their binding to and agglutination of the particles.

As used herein, the phrase "agglutination assay" refers to an investigative procedure for qualitatively assessing and quantitatively measuring the presence, amount, and functional activity of a target entity (analyte) using the agglutination process.

An agglutination assay differs from other assays, for example it differs from coagulation assays such as disclosed in US 2017/0056887 (Hadwen et al.²⁰) as follows:
Agglutination is the process of clumping of particles (solid/ semi-solid or cells). There are many examples of agglutination. For example, hemagglutination is the aggregation of red blood cells, and leukoagglutination is the aggregation of white blood cells.

Coagulation, on the other hand, is the process of a liquid changing to a solid/ semi solid state. An example of coagulation is the process of blood clotting, where blood changes from a liquid to a gel, forming a blood clot. Blood clotting is similar to a gelification process. Clotting has three major steps; i) platelet plug formation, ii) intrinsic or extrinsic pathways, and iii) the common pathway.

The main differences between agglutination and coagulation is that agglutination is the process of particle aggregation while coagulation is the process of the formation of a definitive blood clot. Many particles can agglutinate while only blood can coagulate.

Agglutination is due to an antigen-antibody reaction while coagulation is due to activation of multiple plasma factors.

As used herein, the phrase "agglutination agent" refers to any substance used in an agglutination assay, which can lead to the production of aggregation of particles (agglutinates).

As used herein, the phrase "chemical agglutination agent" refers to a substance that is used in an agglutination assay, which will produce the same effect of particle aggregation but without relying on an antigen-antibody reaction but on other processes that will disrupt the particle suspension and force the particles to collapse to each other and form agglutinates.

**FIG. 1** shows a two (2) plate digital microfluidic device for use in the present disclosure for producing agglutination in liquid analyte samples being tested for the presence or absence of a particular analyte. The major steps using the DMF device are depicted in the three (3) panels starting from the left most panel to the right most panel. In the DMF device the steps of an agglutination assay on the DMF device starts with the left most panel labelled (i) showing one or more samples containing analytes of interest is loaded onto the DMF device which contains agglutinating agents, the middle panel labelled (**ii**) showing the sample being metered into sub-samples and then each sub-sample is mixed with an agglutinating agent for a pre-determined period of time and the right most panel labelled (**iii**) show that the agglutination is observed on the DMF device visually or by camera.

Thus, broadly speaking, the present disclosure provides a method of characterizing a sample containing analytes using a two-plate electrowetting digital microfluidic device (DMF) having a plurality of driving electrodes. The two-plate configuration of the DMF device, allows droplets to be dispensed, split and merged. In a one-plate DMF device higher forces / voltages are required to split and dispense a droplet - even above the dielectric breakdown of the dielectric. In addition, the two-plate DMF device is ideal for imaging the droplet and the contents of the droplet because the majority of the droplet appears as a flat area, while in a one-plate DMF device the curvature of the droplet does not allow for the same ease of imaging. Furthermore, in a one-plate DMF device the area of the droplet exposed to air is larger causing the droplet to evaporate faster which can interfere with the assay.

The method involves loading a fluid sample containing the analytes being tested for, which may contain a surfactant and an agglutination agent which may contain a surfactant on a preselected number of the driving electrodes of DMF device, followed by using electrowetting for bringing the fluid sample in contact with the agglutination agent for agglutination of the analytes to produce an agglutinate, and then characterizing an amount of agglutination of the analytes caused by the agglutination agent.

In an embodiment the fluid sample and/or agglutination agent may contain a surfactant. A surfactant may be used to reduce non-specific binding of analytes to the top or bottom plate of the DMF device. It may also be used to improve fluid sample or agglutination agent movement on the DMF device.

In an embodiment the surfactant is in a pre-dried form, and when it is, the method further includes coating one or more driving electrodes with the pre-dried form of surfactant, either in pre-determined spots or coated across the entire device surface, such that when the fluid sample comes into contact with the pre-dried surfactant, it becomes solubilized, such that the surfactant is present in an amount of at least 0.01% wt:wt in the fluid. In another embodiment agglutination agent is a liquid agglutination agent loaded and metered to preselected driving electrodes.

In a preferred embodiment the method includes a step of actively mixing the agglutination agent with the fluid using electrowetting on the DMF device which advantageously speeds up the process of agglutination when the analyte being tested for is present.

The surfactant may be an ionic surfactant or a non-ionic surfactant depending on the analyte being tested for. Ionic surfactants include but are not limited to sodium dodecyl sulfate, sodium stearate, cetrimonium bromide, cetrimonium chloride, and sodium lauryl sulfate. Non-ionic surfactants include but are not limited to alkylphenol hydroxypolyethylenes (e.g. Triton X RTM), polysorbates (e.g. Tween RTM), poloxamines (e.g. Tetronic RTM), poloxamers (e.g. Pluronic RTM), and sorbitan esters. The choice of ionic or non-ionic surfactants to use is predicated on the type of agglutination assay that is to be performed and type of sample being analyzed. A screening of surfactants for specific assays and sample types should be performed to determine surfactant compatibility.

For example, non-ionic surfactants are preferred when testing blood to determine the blood type as will be discussed in the Example below. Non-ionic surfactants are used with blood to maintain an isotonic environment for the cells, preventing cell lysis.

The agglutination agent may comprise any one or combination of substances capable of producing an agglutinate. Examples of substances include chemical agglutination agents and biological agglutination agents. For agglutination of red blood cells a chemical agglutination agent may be selected from substances such as polycations, including but not limited to poly-L-lysine hydrobromide, poly(dimethyl diallyl ammonium) chloride (e.g. Merquat^{®}-100 RTM, Merquat^{®}-280 RTM, Merquat^{®}-550 RTM), poly-L-arginine hydrochloride, poly-L-histidine, poly(4-vinylpyridine), poly(4-vinylpyridine) hydrochloride, poly(4-vinylpyridine)crosslinked, methyl chloride quaternary salt, poly(4-vinylpyridine-co-styrene); poly(4-vinylpyridinium poly(hydrogen fluoride)); poly(4-vinylpyridinium-P-toluenesulfonate); poly(4-vinylpyridinium-tribromide); poly(4-vinylpyrrolidone-co-2-dimethylaminoethyl methacrylate); poly vinylpyrrolidone, cross-linked; poly vinylpyrrolidone, poly(melamine-co-formaldehyde); partially methylated; hexadimethrine bromide; poly(Glu, Lys) 1:4 hydrobromide; poly(Lys, Ala) 3:1 hydrobromide; poly(Lys, Ala) 2:1 hydro-bromide; poly-L-lysine succinylated; poly(Lys, Ala) 1:1 hydrobromide; and poly(Lys, Trp) 1:4 hydrobromide. The most preferred polycation is poly (dimethyl diallyl ammonium) chloride.

Chemical agglutination agents are used to cause agglutination of any red blood cells, therefore they can be used as a positive control for the blood agglutination assays and they can also be used to agglutinate red blood cells for the determination of hematocrit level as will be discussed in the Example below.

A biological agglutination agent is any substance of biological nature capable of producing an agglutinate. For agglutination of red blood cells, examples include proteins such as lectins (proteins that are able to reversibly bind saccharide structures) and antibodies (e.g. Anti-A, Anti-B, Anti-D), viruses (e.g. influenza virus), antigens, DNA, RNA and DNA or RNA based aptamers. Biological agglutination agents are used to determine the presence or absence of a specific analyte of interest on the red blood cells or in the sample. For example, the antibody Anti-A is used to detect the presence or absence of antigen A on the surface of the red blood cells. As another example influenza virus is used to determine the amount of antibodies against the virus that are present in the plasma and determine the level of immunity of the patient's sample.

The step of characterizing an amount of agglutination of the analytes caused by the agglutination agent is by visual/optical characterization. Other methods of characterization that have been reported include use of electrochemical (e.g. impedance spectroscopy), absorbance and turbidimetric techniques. However, the implementation of these techniques requires additional hardware equipment and several modifications on the DMF device, so that the present process using visual characterization either by an operator visually inspecting the result of the agglutination reaction or using a camera is quite advantageous in that no additional modifications to the system are needed.

The visual characterization can, in examples not falling under the scope of the invention, be by a person visually observing the DMF device to approximate the amount of agglutination. According to the invention, the step of visual characterization is performed using a camera. Non-limiting examples of cameras that could be used include webcams, cell phone cameras, digital camera (including digital single-lens reflex camera, DSLR), video cameras, surveillance cameras point and shoot cameras, cameras with CCD detectors, cameras with CMOS detectors, monochrome cameras, black and white cameras, color cameras. For visual/optical characterization involving a camera, droplet agglutination assessment on DMF (DAAD) was developed. DAAD is an image analysis algorithm used to automatically detect agglutinates in droplets on a DMF device. The DAAD algorithm may be stored in a microprocessor associated with the camera or they may be stored on the microprocessor that is connected to the DMF power supply that controls the driving electrodes of the DMF device or may be stored on a remote computer. The DAAD algorithm may be executed by the microprocessor or the computer.

In an embodiment the agglutination agent comprises particles coated with the agglutination agent. Non-limiting examples include polymer (e.g. latex), gold, silver, nano- and micro- particles. Depending on the analyte of interest, particles are coated with agglutination agents. For example, for the detection of an antigen, particles are coated with an antibody or other agent capable of capturing the antigen of interest. In the case of detection of an antibody, particles should be coated with an antigen or other agent capable of capturing the antibody of interest.

The method may be used for agglutination of a suspension of polymer particles. Non-limiting examples include coated latex particles for Rubella antibody detection,²¹ latex particles coated with an antibody for detection of any virus,²² latex particles coated with streptolysin O,²³ latex particles coated with antibodies for C-reactive protein detection,²⁴ coated latex particles for identification of *Staphylococcus aureus*,²⁵ and coated latex particles for identification of any type of bacteria.

The method disclosed herein may be used for agglutination of a suspension of nanoparticles. Non-limiting examples include nanoparticles coated with an antibody for detection of an antigen, and nanoparticles coated with an antigen for detection of a specific antibody.

The method can be used for agglutination of a suspension of red blood cells to determine the blood type of a patient as will be discussed in the Example below. In this application, the fluid is a blood sample comprising at least just red blood cells. These blood cells are mixed with a liquid diluent such as, but not limited to, plasma, isotonic buffer solution (e.g. phosphate buffered saline (PBS)), a solution containing PBS and serum albumin (e.g. human serum albumin, bovine serum albumin).

However, it will be appreciated that other types of blood samples can be characterized using this method, including whole blood (white and red cells, platelets and plasma), may also include diluted blood (a portion of the whole is taken and mixed it with something else to give some examples), suspension of white blood cells, serum, and plasma.

The method can be used for agglutination of a suspension of red blood cells to determine the hematocrit level as will be discussed in the Example below. In this application, the fluid is a blood sample comprising at least red blood cells. These blood cells may be mixed with a liquid diluent. In this example, any agglutination agent can be used to determine the hematocrit level.

Other types of fluid samples can be characterized using this method, including a virus suspension in a liquid diluent such as, but not limited to, whole blood, serum, plasma, isotonic buffer solution (e.g. PBS), a solution containing PBS and serum albumin (e.g. human serum albumin, bovine serum albumin), nasal mucus, nasopharyngeal mucus, urine and saliva. These fluid samples may be mixed with an agglutinating agent for virus detection.

Other types of fluid samples can be characterized using this method, including a suspension of eukaryotic cells of any other type. The agglutination agent may be any substance capable of causing agglutination of cells.

### EXAMPLES

Non-limiting and exemplary examples of the method disclosed herein will now be discussed but it will be appreciated the present disclosure is not limited to these examples. The invention is solely limited by the appended claims.

### EXAMPLE 1

The first example, illustrated in **FIG. 2****,** is a blood typing assay that uses blood agglutinating antibodies to cause agglutination of red blood cells. A set of 3 antibodies (monoclonal or polyclonal) - specific to antigens A (Anti-A), B (Anti-B) and RhD (Anti-D) as well as a blend of A and B (Anti-A,B) - are used to determine the ABO and Rhesus (Rh) blood types. In the example shown in **FIG. 2****,** the agglutinating reagents were loaded into the device in solution-form; we have also demonstrated analogous methods in which the agglutination reagents are pre-loaded onto the device as dried spots, which become solubilized upon exposure to sample or another reagent (e.g. a dissolution buffer). The complete assay requires just a few minutes and is completely automated.

In examples where pre-dried reagents were used, reconstitution was performed as described by Foley et al.18 in US 2014/0141409 A1.

The assay relies on a phenomenon known as hemagglutination. According to the U.S. National Library of Medicine, haemagglutination (or hemagglutination) is defined as "The aggregation of erythrocytes by agglutinins, including antibodies, lectins, and viral proteins".²⁶ Traditionally, haemagglutination assays have been used to detect variations or polymorphisms of surface markers found on the red blood cell membranes (antigens) to classify the blood in categories (blood groups). There are currently 339 authenticated blood group antigens, 297 of which fall into one of 33 blood group systems. Among these blood group systems, the ABO and Rh are the most well-known systems because of their importance in transfusion medicine.

The ABO system, particularly, is unique because it is the only blood group system in which when antigens are not presented on the red blood cells' surfaces; rather, the reciprocal antibodies are consistently and predictably found as soluble entities in the plasma. ABO antigens are often called histo-blood group antigens as their wide distribution means that they can often be used as histocompatibility antigens as well. Most importantly, the wide distribution of Anti-A and Anti-B makes transfusion of different blood types catastrophic, as haemolytic transfusion reaction (HTR) can cause hyperacute rejection of incompatible kidney, liver and heart transplants. Likewise, the Rh antigens are often used to prevent the haemolytic disease of the fetus and newborn (HDFN).^{27,28}

The assays described in this example are implemented in blood samples; a related test can be performed in serum, which is commonly referred as reverse typing. In reverse typing a patient's serum is mixed with red blood cells with known surface antigens (e.g. A cells and B cells for ABO) and the observation of haemagglutination indicates the presence or absence of the corresponding antibody.²⁹ In either format (normal or "reverse") there is a strong motivation for the development of novel, rapid, and easy to perform blood typing assays as evidenced by the global market for blood typing, which is expected to hit $2.5 billion USD by 2022.³⁰

### EXAMPLE 2

Another application of the new platform is the use of the system for blood donor-recipient crossmatching, a critical operation that must be performed rapidly on-site in high-stake settings such as the emergency room or the trauma-care laboratory (where time is of essence). Specifically, the first step of plasma donation in this setting is to determine the type of the recipient according to the ABO/Rh system, to be able to identify the type of the donor (e.g., B+ donors for a B+ recipient). But this level of selectivity is not sufficient, as there are many other sub-types that can cause incompatibilities that are not captured by the ABO/Rh system, such that a second step is typically performed (often at the patient's bedside, immediately prior to transfusion), in which plasma from a potential donor is tested directly for agglutination with patient blood. An example of a mock crossmatching test executed by DMF hemagglutination and analyzed by DAAD is shown in **FIG. 6****.** In this example, two of four potential donors were found to be compatible with a potential recipient.

### EXAMPLE 3

Agglutination of red blood cells can be used for the determination of hematocrit of blood samples. **FIG. 5** is a drawing showing the result of a DMF agglutination assay for the determination of hematocrit level. In the left of the **FIG. 5** four droplets are shown with different hematocrit levels (ratio of the volume of red blood cells to the total volume of blood) - 20% (top), 40 % (second from top), 60% (second from bottom), 80% (bottom). The droplets were mixed with a chemical agglutination agent that causes non-specific agglutination of red blood cells. The higher the hematocrit level the bigger the agglutinated spot will be. The hematocrit level can be estimated by naked eye or determined using a digital camera. On the right, is shown the output of a processed image captured with a digital camera. The difference in the intensity of the pixels can be used to determine the hematocrit level of the sample.

In particular for the hematocrit determination the integral of the pixel intensities found (inclusively) between a fraction of the total number of pixels is defined as the 'hematocrit score'. [**FIG. 7** In initial experiments, hematocrit scores from a training set of diluted blood samples with artificially defined hematocrit levels between 20 and 60% were found and plotted as a function of hematocrit level and fitted with a second order polynomial: y= -0.0249x2 + 1.092x + 107.3.] Each droplet's hematocrit score was compared to the calibration plot to determine the predicted % hematocrit. (**FIG. 8**).

**FIG. 7** shows the result of a DMF agglutination assay for the determination of hematocrit level. In the left of **FIG. 7** five droplets are shown with different hematocrit levels (ratio of the volume of red blood cells to the total volume of blood) - 60% (top), 50 % (second from top), 40 % (third from top), 30% (second from bottom), 20% (bottom). Similar to **FIG. 5****,** the droplets were mixed with a chemical agglutination agent that causes non-specific agglutination of red blood cells. The higher the hematocrit level the bigger the agglutinated spot will be.

**FIG. 8** shows a calibration curve of hematocrit scores as a function of known hematocrit levels. The markers are experimental data, and error bars represent ±1 standard deviation for n=3 experiments per condition. The dashed second order polynomial was fitted to the data, with R² = 0.9990.

**FIG. 9** shows a bar graph of hematocrit measurements collected from 12 finger-prick whole blood samples from volunteers using the gold standard (left) and the DMF-DAAD method (right). For the set of 12 samples comparison between gold standard and DMF-DAAD methods yields p ≥ 0.5045.

### EXAMPLE 4

The fourth example of the present disclosure illustrated in **FIG. 3****,** is a Latex Immunoagglutination Assay (LIA), which uses a suspension of latex particles to detect an analyte of interest. In the absence of analyte, beads are suspended as individual units and the suspension appears "smooth" (i.e., with no heterogeneous clumps), while in the presence of analyte, the particles aggregate, forming heterogeneous agglutinates that are visible by eye. These assays have widespread utility, such as the detection of mono- and polyvalent antigens, proteins, drugs, steroid hormones, and even micro-organisms.³¹ LIAs are commonly used by clinicians for influenza detection,³² and antibiotic susceptibility testing.³³ For the latter, there is great interest in being able to distinguish between strains of bacteria are or are not antibiotic resistant, to determine which therapy to prescribe. For example, the leading cause of infections acquired in hospitals is Methicillin-resistant Staphylococcus aureus³⁴ (MRSA); clearly, it is a waste of time and resources to prescribe methicillin to patients infected with MRSA. As a proof of principle, we developed latex bead-based agglutination assays on DMF for the detection of Methicillin resistance and susceptibility in strains of bacteria, as featured in **FIG. 3****.**

In this example, a susceptible- (first sample - the pair of droplets on the left) and resistant-strain (second sample - the pair of droplets on the right) of bacteria were mixed with latex beads coated with penicillin-binding protein 2 (PBP2) antibodies (monoclonal or polyclonal) (the left droplet in each pair). Each sample was also mixed with latex beads coated with antibodies not specific for PBP2 which acts as a negative control (the right droplet in each pair). The results indicate that the first sample shows weak agglutination indicating susceptibility (suggesting that patients infected with these bacteria might be treatable with methicillin), and the second sample shows strong agglutination (suggesting that patients infected with these bacteria should receive alternate treatments). In sum, the DMF based assay allows for rapid detection of three states: no agglutination (for the negative controls), weak agglutination for antibiotic susceptible bacteria, and strong agglutination for antibiotic resistant bacteria) to be easily identified by eye.

### Visual Determination of Agglutination Results

The simplest mode of detection for agglutination assays is observation by eye by the user; this method works well for the examples that we have reduced to practice, described above. But agglutination is also amenable to complete automation via image processing. There have been several reports of automated detection of haemagglutination in fluidic channels, but they rely on ancillary equipment (e.g. microscope,⁵ waveguide,³⁵ etc.) and complicated post processing procedures. For example, Huet et al.⁵ trained an artificial network in MATLAB to detect the progress of agglutination, but the algorithms are not universal, and a new training set is required for each new imaging setup.

In contrast, the present method as depicted in **FIGS. 4A** and **4B****,** is straightforward and can be applied to any system with a digital camera. For optical characterization involving a camera, droplet agglutination assessment on DMF (DAAD) is performed in 8 steps. The first six image pre-processing steps (i - vi) are the same for blood typing (**FIG. 2**), latex agglutination assays (**FIG. 3**), hematocrit analysis (**FIG. 5**) and donor compatibility testing (**FIG. 6**).

FIG. 4A**-I** shows step i) in which a camera is used to collect an image of the device. The camera is positioned at an angle relative to the plane perpendicular to the DMF device. Images are typically captured at the maximum resolution of each camera (but images at lower resolutions can be processed as well).

**FIG. 4A****-ii** shows step ii in which the image is corrected for perspective by defining four coordinates in the source image and four reference coordinates. A 3×3 matrix is calculated based on each set of coordinates (image - reference corresponding pair) and then the same matrix is applied to the source image to acquire the perspective-corrected image.

**FIG. 4A****-iii** shows **step iii**) in which the center of the DMF device is located automatically by detecting known device features, and this region of the image is isolated for further processing.

**FIG. 4A****-iv** shows **step iv** in which the droplets are detected by identifying contours and combining neighbouring contours to form a rectangular region of interest (ROI) for each droplet which is used to define a mask to extract an image.

**FIG. 4A****-v** shows **step v** in which the ROI image corresponding to each droplet is masked, isolated and converted from RGB to grayscale.

**FIG. 4B****-left** shows **step vi** where each isolated image is flattened into a one-dimensional array and normalized such that the pixel intensities cover the full 8-bit range [0-255], and then sorted by pixel-value from lowest to highest.

**FIG. 4B****-right** shows **step vii** in which the slope of pixel intensity in this gradient is then used as an indication of the degree of agglutination (process used in blood typing, donor compatibility tests, step viii). For example, in **FIG. 4B** the steep slope of the pixels' intensities for A, D (Rh), and A,B blend indicate agglutination, while the flat slope for B indicates no agglutination. We have developed similar image processing methods to automate the detection of the agglutination of latex beads (**FIG. 3**), highlighting the flexibility of this method compared to previous reports.⁵

Within the visual characterization, other methods of analysis could be performed. Three alternative agglutination detection algorithms were tested and compared to DAAD: the histogram method, the standard deviation method and the variance method. In the histogram method, DAAD sub-steps (i) - (v) were performed to isolate each ROI image. For each image, a histogram was generated from the number of pixels for each pixel intensity value. The histogram was smoothed with a moving average filter (window = 10 bins), and in the smoothed dataset, the major peaks were identified by finding local maxima by comparison of pixel intensities with neighbouring values.

The average pixel intensity of the major peaks in the smoothed histogram was defined as the threshold T. Finally, the agglutination score was defined as 100×(S>T/S), where S is the number of pixels in the ROI image and S>T is the number of pixels with intensity greater than T. In the standard deviation method (adapted from previous reports³⁶), DAAD sub-steps (i) - (vi) were performed, after which the array was normalized (again) to the range [0,1], and the standard deviation a of pixel intensities was defined as the agglutination score. In the variance method (adapted from previous ᵣeports^{5,6},³⁷), DAAD sub-steps (i) - (v) were performed to isolate each ROI image. The local variance of each pixel relative to its neighbors _{UP}² was calculated using a 3×3 matrix, and the average variance of all the pixels in the image ${{σ}_{p}^{2}}_{¯}$ was determined.

The agglutination score was defined as $100 \times {σ}_{p}^{2}$. A series of 86 samples (344 ROIs) were evaluated by DAAD and the three alternate methods. The 'best' agglutination thresholds (with the highest true positive rate and the lowest false positive rate) for the alternate methods were found to be 10 a.u., 0.13 a.u., and 1 a.u., for the histogram method, standard deviation method, and the variance method, respectively (**FIG. 10**).

In summary, inventors report the first two-plate digital microfluidic system and method capable of carrying out agglutination assays. The inventors have demonstrated four non-limiting and exemplary embodiments of this invention in the four Examples above. The first embodiment is a blood typing haemagglutination assay - the first that we are aware of to be implemented on a two-plate DMF device. This method was demonstrated to be compatible with the use of solution-phase or dried agglutinating antibodies, which are mixed with whole, undiluted blood, with results determined by eye within minutes. As an extension to the blood typing assay, when the blood sample is mixed with prospective donor samples donor compatibility testing can be performed, to indicate the right donor for the recipient patient (second embodiment). In addition, to the above assays we demonstrate the use of hemagglutination, using a chemical reagent for the determination of the samples' hematocrit (third embodiment). In the fourth embodiment, we implemented a DMF method for carrying out latex immunoagglutination assays (LIAs). In this example, a test was demonstrated for antibiotic susceptibility; but we anticipate that any LIA should be compatible. Finally, we report an imaging-based readout with custom but generalizable algorithm for interpreting the results of DMF agglutination assays. When considered together, a user could load samples, press a button, and receive results in a matter of minutes.

**Table 1** below is a table that outlines some of the significant differences between our new method reported here and the two previous DMF agglutination methods reported in the literature.

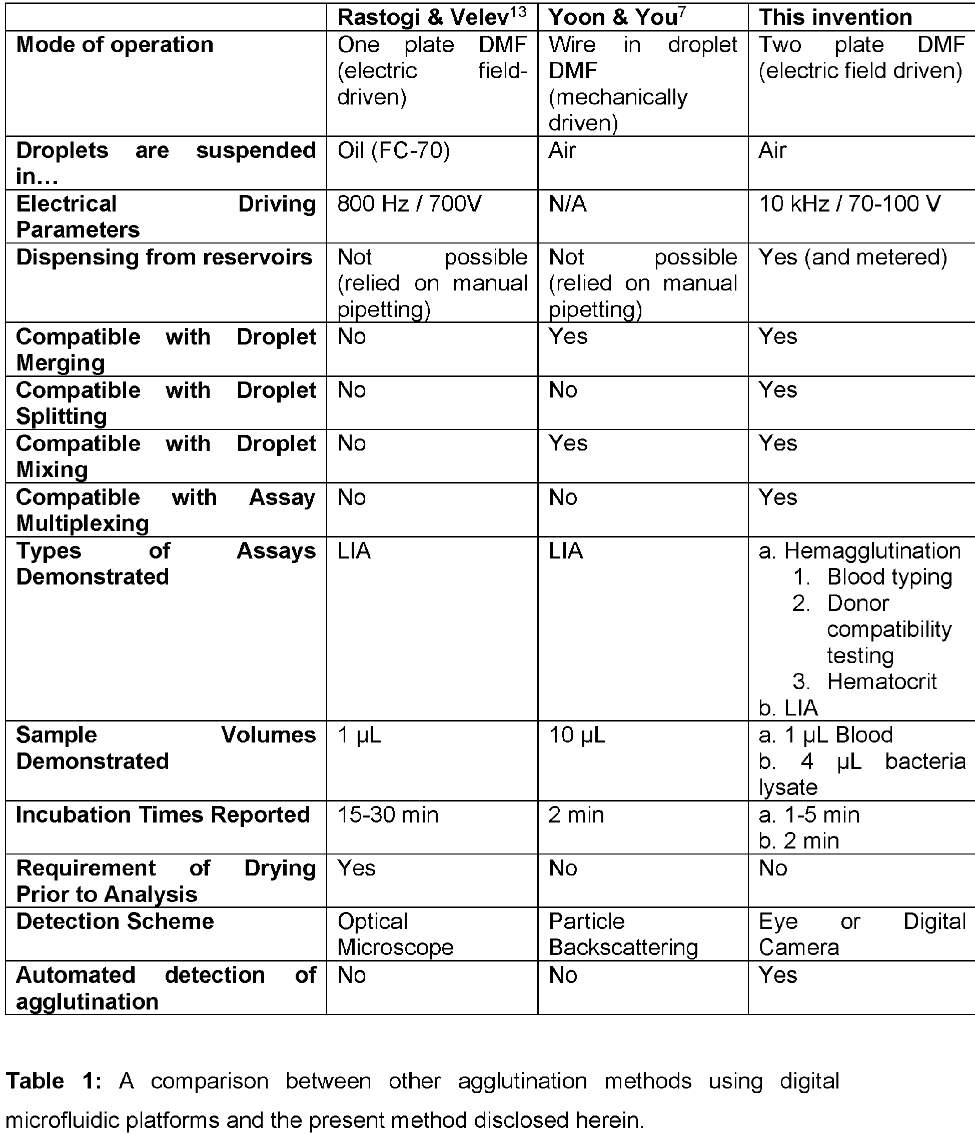

The present inventors are aware of literature reports demonstrating agglutination assays using digital microfluidics but all of them are irrelevant to the current invention.^{7,13} None of the previous reports has used a two-plate electrowetting device to perform agglutination assays. In contrast, a different type of assays, coagulation assays²⁰ and plasma separation using lectins³⁸ have been demonstrated on two-plate DMF devices but none of the above is relevant to the present invention. In addition, the detection of agglutination is performed either by naked eye or using the DAAD (our unique detection algorithm which detects agglutination in images captured with a digital camera). The present method does rely on using absorbance modules to determine agglutination as previously reported³⁹ and the algorithm is not using any of the previously reported methods for the detection of agglutination as these methods rely on expensive imaging equipment (microscope setups or high-end DSLR cameras)^{7,13} and depend highly on the imaging conditions (brightness, contrast, white balance, etc.). The performance of some other previously reported methods was compared to DAAD and it has been shown herein that the present DAAD outperformed all of them.

In summary, in one aspect the present disclosure provides a method of characterizing a sample containing analytes using a two-plate electrowetting digital microfluidic device (DMF) having a plurality of driving electrodes. The method comprises the steps of loading a fluid sample containing the analytes and a surfactant and an agglutination agent on said DMF device; and using electrowetting for bringing the fluid sample in contact with the agglutination agent for agglutination of the analytes with the agglutination agent.

In another aspect the present disclosure provides a two-plate electrowetting DMF device, comprising a first plate, a second plate spaced from said first plate, one of said first and second plates having a plurality of driving electrodes; and a surface on either the first plate or the second plate having a surfactant in a pre-dried form coating the surface in preselected locations, another surface on either the first plate or the second plate having an agglutination agent in a pre-dried from coating the other surface in preselected locations.

The surface coated by the surfactant and the surface coated with the agglutination agent are either different or the same.

The present disclosure also provides a kit, comprising a two-plate electrowetting digital microfluidic device (DMF) having a plurality of driving electrodes; a surfactant for placement on one of said two plates; and an agglutination agent for placement of one of the two plates.

The specific embodiments described above have been shown by way of example, and it should be understood that these embodiments may be susceptible to various modifications and alternative forms. It should be further understood that the claims are not intended to be limited to the particular forms disclosed, but rather to cover all modifications, equivalents, and alternatives falling within the scope of this disclosure.

### References

1. Castro, D., Conchouso, D., Kodzius, R., Arevalo, A. & Foulds, I. G. High-throughput incubation and quantification of agglutination assays in a microfluidic system. Genes 9, 281 (2018).
2. Ma, Z. et al. Homogeneous agglutination assay based on micro-chip sheathless flow cytometry. Biomicrofluidics 9, 066501-066501 (2015).
3. Lucas, L. J., Han, J.-H., Chesler, J. & Yoon, J.-Y. Latex immunoagglutination assay for a vasculitis marker in a microfluidic device using static light scattering detection. Biosens Bioelectron 22, 2216-2222 (2007).
4. Afshar, R., Moser, Y., Lehnert, T. & Gijs, M. A. M. Three-dimensional magnetic focusing of superparamagnetic beads for on-chip agglutination assays. Anal. Chem. 83, 1022-1029 (2011).
5. Huet, M., Cubizolles, M. & Buhot, A. Real time observation and automated measurement of red blood cells agglutination inside a passive microfluidic biochip containing embedded reagents. Biosens Bioelectron 93, 110-117 (2017).
6. Huet, M., Cubizolles, M. & Buhot, A. Red Blood Cell Agglutination for Blood Typing Within Passive Microfluidic Biochips. High-throughput 7, 10 (2018).
7. Yoon, J.-Y. & You, D. J. Backscattering particle immunoassays in wire-guide droplet manipulations. Journal of biological engineering 2, 15-15 (2008).
8. Kirby, A. E. & Wheeler, A. R. Digital microfluidics: an emerging sample preparation platform for mass spectrometry. Anal. Chem. 85, 6178-6184 (2013).
9. Rackus, D. G. et al. A digital microfluidic device with integrated nanostructured microelectrodes for electrochemical immunoassays. Lab Chip 15, 3776-3784 (2015).
10. Ng, A. H. et al. Digital microfluidic platform for the detection of rubella infection and immunity: a proof of concept. Clin. Chem. 61, 420-429 (2015).
11. Ng, A. H. C. et al. A digital microfluidic system for serological immunoassays in remote settings. Sci. Transl. Med. 10, eaar6076 (2018).
12. Jebrail, M. J. et al. Combinatorial Synthesis of Peptidomimetics Using Digital Microfluidics. J. Flow Chem. 2, 103-107 (2012).
13. Rastogi, V. & Velev, O. D. Development and evaluation of realistic microbioassays in freely suspended droplets on a chip. Biomicrofluidics 1, 14107 (2007).
14. Holmes, H. R. & Böhringer, K. F. Transporting droplets through surface anisotropy. Microsystems & Nanoengineering 1, 15022 (2015).
15. Darhuber, A. A., Valentino, J. P., Davis, J. M., Troian, S. M. & Wagner, S. Microfluidic actuation by modulation of surface stresses. Appl. Phys. Lett. 82, 657-659 (2003).
16. Li, A. et al. Programmable droplet manipulation by a magnetic-actuated robot. Science Advances 6, eaay5808 (2020).
17. Ding, X. et al. Surface acoustic wave microfluidics. Lab on a Chip 13, 3626-3649 (2013).
18. Foley, J., Burde, S., Pamula, V. K. & Pollack, M. G. Reagent storage on a droplet actuator. (2017).
19. Stavitsky, A. B. in Encyclopedia of Immunology (Second Edition) (ed. Delves, P. J.) 56-59 (Elsevier, 1998).
20. Hadwen, B. J. et al. Droplet microfluidic device and methods of sensing the results of an assay therein. (2017).
21. Freeman, S., Clark, L. & Dumas, N. Evaluation of a latex agglutination test for detection of antibodies to rubella virus in selected sera. J Clin Microbiol 18, 197-198 (1983).
22. Kasempimolporn, S., Saengseesom, W., Lumlertdacha, B. & Sitprija, V. Detection of rabies virus antigen in dog saliva using a latex agglutination test. J Clin Microbiol 38, 3098-3099 (2000).
23. Kotby, A. A., Habeeb, N. M. & Elarab, El, S. E. Antistreptolysin O titer in health and disease: levels and significance. Pediatric reports 4, (2012).
24. Winkles, J., Lunec, J. & Deverill, I. Enhanced-latex-agglutination assay for C-reactive protein in serum, with use of a centrifugal analyzer. Clin. Chem. 33, 685-689 (1987).
25. Idelevich, E. A. et al. Bacteriophage-based latex agglutination test for rapid identification of Staphylococcus aureus. J Clin Microbiol 52, 3394-3398 (2014).
26. Hemagglutination. Available at: https://www.ncbi.nlm.nih.gov/mesh/68006384. (Accessed: 28 February 2019)
27. Daniels, G. Human blood groups: introduction. Human blood groups 1-10 (2013).
28. Dean, L. Blood groups and red cell antigens. (2005).
29. Blood Grouping Reagents. (Beckman Coulter Technical Document). Available at: http://www.mycts.org/Portals/O/Assay_PI/WholeBlood/ABORH.pdf. (Accessed: 28 February 2019)
30. Global Blood Group Typing Market. (Transparency Market Research). Available at: https://www.transparencymarketresearch.com/pressrelease/blood-group-typing-market.htm. (Accessed: 28 February 2019)
31. Osada, Y., Ping Gong, J. & Tanaka, Y. Polymer Gels. Journal of Macromolecular Science, Part C: Polymer Reviews 44, 87-112 (2004).
32. Chen, J. et al. A latex agglutination test for the rapid detection of avian influenza virus subtype H5N1 and its clinical application. J Vet Diagn Invest 19, 155-160 (2007).
33. van Griethuysen, A. et al. Rapid slide latex agglutination test for detection of methicillin resistance in Staphylococcus aureus. J Clin Microbiol 37, 2789-2792 (1999).
34. Nemr, C. R. et al. Nanoparticle-Mediated Capture and Electrochemical Detection of Methicillin-Resistant Staphylococcus aureus. Anal. Chem. 91, 2847-2853 (2019).
35. Ashiba, H. et al. Hemagglutination detection for blood typing based on waveguide-mode sensors. Sensing and Bio-Sensing Research 3, 59-64 (2015).
36. Castro, D., Conchouso, D., Kodzius, R., Arevalo, A. & Foulds, I. G. High-Throughput Incubation and Quantification of Agglutination Assays in a Microfluidic System. Genes 9, 281 (2018).
37. Kline, T. R., Runyon, M. K., Pothiawala, M. & Ismagilov, R. F. ABO, D Blood Typing and Subtyping Using Plug-Based Microfluidics. Anal. Chem. 80, 6190-6197 (2008).
38. Sista, R. S. et al. Digital Microfluidic Platform to Maximize Diagnostic Tests with Low Sample Volumes from Newborns and Pediatric Patients. Diagnostics 10, 21 (2020).
39. Srinivasan, V., Pamula, V. K., Pollack, M. G. & Fair, R. B. Droplet-based affinity assays. (2013).

## Claims

1. A method of characterizing a sample to determine a presence or absence of pre-selected analytes using agglutination assays, comprising steps of:
providing a two-plate electrowetting digital microfluidic device, DMF, having a plurality of driving electrodes;
loading a fluid sample containing the analytes and an agglutination agent capable of causing agglutination onto separate driving electrodes of said DMF device;
using electrowetting for bringing droplets of the fluid sample in contact with the agglutination agent for agglutination of any of the analytes present in the sample with the agglutination agent to produce an agglutinate; and
visually characterizing any agglutinate formed due to the presence of the pre-selected analytes in the fluid sample by:
using a camera positioned at an angle to collect an image of the DMF device,;
performing perspective correction;
locating the center of the DMF device automatically by detecting known device features, and isolating the region of the image for further processing;
detecting the droplets by identifying contours and combining neighbouring contours to form a rectangular region of interest for each droplet;
masking and isolating the region of interest image corresponding to each droplet, and converting for each droplet the region of interest image into gray scale;
flattening each isolated image into a one-dimensional array which is normalized such that pixel intensities cover a full 8-bit range, and sorted by pixel- value from lowest to highest;
using a slope of pixel intensity as an indication of a degree of agglutination; and
determining the degree of agglutination for each of the regions of interest based on a slope of a gradient of the sorted pixel-intensity array.

2. The method according to claims 1, further comprising mixing an ionic or nonionic surfactant with the fluid sample containing the analytes or the agglutination agent, or both, wherein the ionic or non-ionic surfactant is optionally provided in a pre-dried form and wherein said method further comprises coating one or more driving electrodes with the pre-dried form of said surfactant, either in pre-determined spots or coated across the entire array of driving electrodes, such that when the fluid sample comes into contact with the pre-dried surfactant, it becomes solubilized.

3. The method according to claim 2, wherein said ionic surfactants are selected from the group consisting of sodium dodecyl sulfate, sodium stearate, cetrimonium bromide, cetrimonium chloride, and sodium lauryl sulfate and said non-ionic surfactants are selected from the group consisting of alkylphenol hydroxypolyethylenes, polysorbates, poloxamines, poloxamers, and sorbitan esters.

4. The method according to any one of claims 1 to 3, wherein said agglutination agent is a liquid agglutination agent loaded and metered to preselected driving electrodes.

5. The method according to any one of claims 1 to 4, further comprising a step of actively mixing the agglutination agent with the fluid sample using electrowetting on the DMF device.

6. The method according to any one of claims 1 to 5, wherein said agglutination agent comprises of one or more chemical agglutination agents and biological agglutination agents and optionally said chemical agglutination agent is selected from the group consisting of poly-L-lysine hydrobromide, poly(dimethyl diallyl ammonium) chloride, poly-L-arginine hydrochloride, poly-L-histidine, poly(4-vinylpyridine), poly(4-vinylpyridine) hydrochloride, poly(4-vinylpyridine)crosslinked, methyl chloride quaternary salt, poly(4-vinylpyridine-co-styrene); poly(4-vinylpyridinium poly(hydrogen fluoride)); poly(4-vinylpyridinium-P-toluenesulfonate); poly(4-vinylpyridinium-tribromide); poly(4-vinylpyrrolidone-co-2-dimethylaminoethyl methacrylate); poly vinylpyrrolidone, cross-linked; poly vinylpyrrolidone, poly(melamine-co-formaldehyde); partially methylated; hexadimethrine bromide; poly(Glu, Lys) 1:4 hydrobromide; poly(Lys, Ala) 3:1 hydrobromide; poly(Lys, Ala) 2:1 hydro-bromide; poly-L-lysine succinylated; poly(Lys, Ala) 1:1 hydrobromide; poly(Lys, Trp) 1:4 hydrobromide; and poly (dimethyl diallyl ammonium) chloride and said biological agglutination agent is selected from the group consisting of proteins, antibodies, viruses and antigens, DNA, RNA and DNA or RNA based aptamers.

7. The method according to claim 6, wherein said proteins comprise lectins able to reversibly bind saccharide structures, said antibodies comprising Anti-A, Anti-B and Anti-D and said viruses comprise influenza virus.

8. The method according to any one of claims 1 to 7, wherein said agglutination agent comprises particles coated with said agglutination agent, optionally said particles include any one or combination of polymer particles, gold, silver, nano- and micro- particles.

9. The method according to any one of claims 1 to 8**,** for a use of agglutination of a suspension of polymer particles or for a use of agglutination of a suspension of red blood cells.

10. The method according to any one of claims 1 to 8, wherein said fluid is blood comprising at least red blood cells and optionally said agglutination agent is a chemical agglutination agent used to agglutinate red blood cells for the determination of hematocrit level.

11. A system comprising:
a two-plate electrowetting DMF device comprising:
a first plate, a second plate spaced from said first plate, one of said first and second plates having a plurality of driving electrodes; and
a surface on either the first plate or the second plate having a surfactant in a pre-dried form coating the surface in preselected locations, another surface on either the first plate or the second plate having an agglutination agent in a pre-dried from coating the other surface in preselected locations;
a camera; and
means for image processing configured to perform the imaging processing as defined in any one of claims 1 to 10.

12. The system according to claim 11, further comprising a microprocessor connected to a power supply and said plurality of driving electrodes and programmed with instructions to provide power to said driving electrodes in a pre-selected pattern for moving droplets of fluid sample being studied for presence of pre-selected analytes located therein and an agglutination agent over the electrodes.

13. The system according to claim 11 or 12, wherein the surface coated by the surfactant and the surface coated with the agglutination agent are either different or the same.

## Patentansprüche

1. Verfahren zum Charakterisieren einer Probe zum Bestimmen eines Vorhandenseins oder Fehlens von vorausgewählten Analyten unter Verwendung von Agglutinationstests, umfassend folgende Schritte:
Bereitstellen einer digitalen mikrofluidischen Elektrobenetzungsvorrichtung mit zwei Platten, DMF, die eine Vielzahl von Ansteuerelektroden aufweist;
Laden einer Fluidprobe, die die Analyten und ein Agglutinationsmittel, das zum Bewirken von Agglutination in der Lage ist, auf separate Ansteuerelektroden der DMF-Vorrichtung;
Verwenden von Elektrobenetzen, um Tröpfchen der Fluidprobe zur Agglutination eines beliebigen der in der Probe vorhandenen Analyten mit dem Agglutinationsmittel unter Erzeugen eines Agglutinats in Kontakt mit dem Agglutinationsmittel zu bringen; und
visuelles Charakterisieren eines beliebigen aufgrund des Vorhandenseins der vorausgewählten Analyten in der Fluidprobe ausgebildeten Agglutinats durch:
Verwenden einer in einem Winkel positionierten Kamera zum Aufnehmen eines Bildes der DMF-Vorrichtung;
Durchführen einer Perspektivkorrektur;
automatisches Lokalisieren des Zentrums der DMF-Vorrichtung durch Erfassen bekannter Vorrichtungsmerkmale und Isolieren der Region des Bildes zur weiteren Verarbeitung;
Erfassen der Tröpfchen durch Erkennen von Konturen und Kombinieren benachbarter Konturen unter Ausbilden einer rechteckigen Region von Interesse jedes Tröpfchens;
Maskieren und Isolieren des Bildes der Region von Interesse, das jedem Tröpfchen entspricht, und Umwandeln des Bildes der Region von Interesse jedes Tröpfchens in eine Graustufe;
Abflachen jedes isolierten Bildes in ein eindimensionales Array, das derart normalisiert wird, dass Pixelintensitäten einen vollständigen 8-Bit-Bereich abdecken, und nach Pixelwert vom niedrigsten zum höchsten sortiert wird;
Verwenden einer Steigung der Pixelintensität als eine Angabe eines Agglutinationsgrades; und
Bestimmen des Agglutinationsgrades jeder der Regionen von Interesse basierend auf einer Steigung eines Gradienten des sortierten Pixelintensitätsarrays.

2. Verfahren nach Anspruch 1, ferner umfassend Mischen eines ionischen oder nichtionischen Tensids mit der die Analyten oder das Agglutinationsmittel oder beides enthaltenden Fluidprobe, wobei das ionische oder nichtionische Tensid gegebenenfalls in einer vorgetrockneten Form bereitgestellt wird und wobei das Verfahren ferner Beschichten einer oder mehrerer Ansteuerelektroden mit der vorgetrockneten Form des Tensids entweder an vorbestimmten Stellen oder über das gesamte Array von Ansteuerelektroden beschichtet derart umfasst, dass die Fluidprobe löslich gemacht wird, wenn sie mit dem vorgetrockneten Tensid in Kontakt kommt.

3. Verfahren nach Anspruch 2, wobei die ionischen Tenside ausgewählt sind aus der Gruppe, bestehend aus Natriumdodecylsulfat, Natriumstearat, Cetrimoniumbromid, Cetrimoniumchlorid und Natriumlaurylsulfat, und die nichtionischen Tenside ausgewählt sind aus der Gruppe, bestehend aus Alkylphenolhydroxypolyethylenen, Polysorbaten, Poloxaminen, Poloxameren und Sorbitanestern.

4. Verfahren nach einem der Ansprüche 1 bis 3, wobei es sich bei dem Agglutinationsmittel um ein flüssiges Agglutinationsmittel handelt, das auf vorausgewählte Ansteuerelektroden geladen und dosiert wird.

5. Verfahren nach einem der Ansprüche 1 bis 4, ferner umfassend einen Schritt des aktiven Mischens des Agglutinationsmittels mit der Fluidprobe unter Verwendung von Elektrobenetzen auf der DMF-Vorrichtung.

6. Verfahren nach einem der Ansprüche 1 bis 5, wobei das Agglutinationsmittel ein oder mehrere chemische Agglutinationsmittel und biologische Agglutinationsmittel umfasst und das chemische Agglutinationsmittel gegebenenfalls ausgewählt ist aus der Gruppe, bestehend aus Poly-L-lysin-Hydrobromid, Poly(dimethyldiallylammonium)chlorid, Poly-L-arginin-Hydrochlorid, Poly-L-histidin, Poly(4-vinylpyridin), Poly(4-vinylpyridin)-Hydrochlorid, vernetztem Poly(4-vinylpyridin), quaternärem Methylchlorid-Salz, Poly(4-vinylpyridin-Co-Styrol); Poly(4-vinylpyridinium-Poly(hydrogenfluorid)); Poly(4-vinylpyridinium-p-toluolsulfonat); Poly(4-vinylpyridiniumtribromid); Poly(4-vinylpyrrolidon-Co-2-Dimethylaminoethylmethacrylat); vernetztem Polyvinylpyrrolidon; Polyvinylpyrrolidon, Poly(melamin-Co-Formaldehyd); partiell methyliert; Hexadimethrinbromid; Poly(Glu, Lys)-1:4-Hydrobromid; Poly(Lys, Ala)-3:1-Hydrobromid; Poly(Lys, Ala)-2:1-Hydrobromid; succinyliertem Poly-L-lysin; Poly(Lys, Ala)-1:1-Hydrobromid; Poly(Lys, Trp)-1:4-Hydrobromid; und Poly(dimethyldiallylammonium)chlorid, und das biologische Agglutinationsmittel ausgewählt ist aus der Gruppe, bestehend aus Proteinen, Antikörpern, Viren und Antigenen, DNA, RNA und DNA- oder RNA-basierten Aptameren.

7. Verfahren nach Anspruch 6, wobei die Proteine Lektine umfassen, die in der Lage sind, Saccharidstrukturen reversibel zu binden, wobei die Antikörpern Anti-A, Anti-B und Anti-D umfassen und die Viren Influenzaviren umfassen.

8. Verfahren nach einem der Ansprüche 1 bis 7, wobei das Agglutinationsmittel Partikel umfasst, die mit dem Agglutinationsmittel beschichtet sind; wobei die Partikel gegebenenfalls ein beliebiges oder eine Kombination von Polymerpartikeln, Gold-, Silber-, Nano- und Mikropartikeln beinhalten.

9. Verfahren nach einem der Ansprüche 1 bis 8, zur Verwendung einer Agglutination einer Suspension von Polymerpartikeln oder zur Verwendung einer Agglutination einer Suspension von roten Blutkörperchen.

10. Verfahren nach einem der Ansprüche 1 bis 8, wobei es sich bei dem Fluid um Blut handelt, umfassend mindestens rote Blutkörperchen, und es sich bei dem Agglutinationsmittel gegebenenfalls um ein chemisches Agglutinationsmittel handelt, das zum Agglutinieren roter Blutkörperchen zur Bestimmung des Hämatokritwerts verwendet wird.

11. System, umfassend:
eine Elektrobenetzungs-DMF-Vorrichtung mit zwei Platten, umfassend:
eine erste Platte, eine zweite von der ersten Platte beabstandete Platte, wobei eine der ersten und zweiten Platte eine Vielzahl von Ansteuerelektroden aufweist; und
eine Fläche auf entweder der ersten Platte oder der zweiten Platte, wobei die Fläche an vorausgewählten Stellen mit einem Tensid in vorgetrockneter Form beschichtet ist, eine andere Fläche auf entweder der ersten Platte oder der zweiten Platte, wobei die andere Fläche an vorausgewählten Stellen mit einem Agglutinationsmittel in vorgetrockneter Form beschichtet ist;
eine Kamera; und
Mittel zur Bildverarbeitung, die zum Durchführen einer Bildverarbeitung wie in einem der Ansprüche 1 bis 10 definiert konfiguriert sind.

12. System nach Anspruch 11, ferner umfassend einen Mikroprozessor, der mit einer Stromversorgung und der Vielzahl von Ansteuerelektroden verbunden ist und mit Anweisungen zum Bereitstellen von Strom an den Ansteuerelektroden in einem vorausgewählten Muster zum Bewegen von Tröpfchen einer Fluidprobe, die auf Vorhandensein von darin befindlichen vorausgewählten Analyten untersucht wird, und eines Agglutinationsmittels über die Elektroden programmiert ist.

13. System nach Anspruch 11 oder 12, wobei die mit dem Tensid beschichtete Fläche und die mit dem Agglutinationsmittel beschichtete Fläche entweder verschieden oder gleich sind.

## Revendications

1. Procédé de caractérisation d'un échantillon pour déterminer la présence ou l'absence d'analytes présélectionnés à l'aide de dosages d'agglutination, comprenant les étapes de :
fourniture d'un dispositif numérique microfluidique d'électromouillage à deux plaques (DMF) comportant une pluralité d'électrodes motrices ;
chargement d'un échantillon de fluide contenant les analytes ainsi qu'un agent d'agglutination susceptible de provoquer une agglutination sur des électrodes motrices séparées dudit dispositif DMF ;
utilisation de l'électromouillage pour mettre en contact des gouttelettes de l'échantillon de fluide avec l'agent d'agglutination afin d'agglutiner l'un quelconque des analytes présents dans l'échantillon avec l'agent d'agglutination pour produire un agglutinat ; et
caractérisation visuelle d'un quelconque agglutinat formé en raison de la présence des analytes présélectionnés dans l'échantillon de fluide par :
l'utilisation d'une caméra positionnée suivant un angle donné pour collecter une image du dispositif DMF ;
mise en œuvre d'une correction de perspective ;
localisation de manière automatique du centre du dispositif DMF par la détection des éléments connus du dispositif et l'isolement de la région de l'image pour un traitement ultérieur ;
détection des gouttelettes par l'identification des contours et la combinaison des contours voisins pour former une région d'intérêt rectangulaire pour chaque gouttelette ;
masquage et isolement de l'image de la région d'intérêt correspondant à chaque gouttelette, et conversion pour chaque gouttelette de l'image de la région d'intérêt en niveaux de gris ;
aplatissement de chaque image isolée en un tableau unidimensionnel qui est normalisé de sorte que les intensités de pixels couvrent une plage complète de 8 bits, et sont triées par valeur de pixel de la plus basse à la plus élevée ;
utilisation de la pente de l'intensité de pixels comme indication du degré d'agglutination ; et
détermination du degré d'agglutination pour chacune des régions d'intérêt sur la base de la pente d'un gradient du tableau d'intensités de pixels triées.

2. Procédé selon la revendication 1, comprenant en outre le mélange d'un tensioactif ionique ou non ionique avec l'échantillon de fluide contenant les analytes ou l'agent d'agglutination, ou les deux, dans lequel le tensioactif ionique ou non ionique est éventuellement fourni sous une forme pré-séchée et dans lequel ledit procédé comprend en outre le revêtement d'une ou plusieurs électrodes motrices avec la forme pré-séchée dudit tensioactif, soit à des endroits prédéterminés, soit sur l'ensemble du réseau d'électrodes motrices, de sorte que, lorsque l'échantillon de fluide entre en contact avec le tensioactif pré-séché, celui-ci est solubilisé.

3. Procédé selon la revendication 2, dans lequel lesdits tensioactifs ioniques sont choisis dans le groupe constitué par du dodécylsulfate de sodium, du stéarate de sodium, du bromure de cétrimonium, du chlorure de cétrimonium et du laurylsulfate de sodium et lesdits tensioactifs non ioniques sont choisis dans le groupe constitué par des hydroxypolyéthylènes d'alkylphénol, des polysorbates, des poloxamines, des poloxamères et des esters de sorbitan.

4. Procédé selon l'une quelconque des revendications 1 à 3, dans lequel ledit agent d'agglutination est un agent d'agglutination liquide chargé et mesuré à l'aide des électrodes motrices présélectionnées.

5. Procédé selon l'une quelconque des revendications 1 à 4, comprenant en outre une étape de mélange actif de l'agent d'agglutination avec l'échantillon de fluide à l'aide d'un électromouillage sur le dispositif DMF.

6. Procédé selon l'une quelconque des revendications 1 à 5, dans lequel ledit agent d'agglutination comprend un ou plusieurs agents d'agglutination chimiques et agents d'agglutination biologiques, et, éventuellement, ledit agent d'agglutination chimique est choisi dans le groupe constitué par du bromhydrate de poly-L-lysine, du polychlorure de diméthyldiallylammonium, du chlorhydrate de poly-L-arginine, de la poly-L-histidine, de la poly(4-vinylpyridine), du chlorhydrate de poly(4-vinylpyridine), de la poly(4-vinylpyridine) réticulée, du sel quaternaire de chlorure de méthyle, du poly(4-vinylpyridine-co-styrène) ; du poly(4-vinylpyridinium poly(fluorure d'hydrogène)) ; du poly(P-toluènesulfonate de 4-vinylpyridinium) ; du poly(tribromure de 4-vinylpyridinium) ; du poly(4-vinylpyrrolidone-co-méthacrylate de 2-diméthylaminoéthyle) ; de la polyvinylpyrrolidone réticulée ; de la polyvinylpyrrolidone, poly(mélamine-co-formaldéhyde) ; partiellement méthylée ; du bromure d'hexadiméthrine ; du bromhydrate de poly(Glu, Lys) 1:4 ; du bromhydrate de poly(Lys, Ala) 3:1 ; du bromhydrate de poly(Lys, Ala) 2:1 ; de la poly-L-lysine succinylée ; du bromhydrate de poly(Lys, Ala) 1:1 ; du bromhydrate de poly(Lys, Trp) 1:4 ; et du chlorure de poly(diméthyldiallyl-ammonium), et ledit agent d'agglutination biologique est choisi dans le groupe constitué par des protéines, des anticorps, des virus et des antigènes, de l'ADN, de l'ARN et des aptamères à base d'ADN ou d'ARN.

7. Procédé selon la revendication 6, dans lequel lesdites protéines comprennent des lectines susceptibles de se lier de manière réversible à des structures saccharidiques, lesdits anticorps comprenant des Anti-A, Anti-B et Anti-D et lesdits virus comprennent le virus de la grippe.

8. Procédé selon l'une quelconque des revendications 1 à 7, dans lequel ledit agent d'agglutination comprend des particules revêtues dudit agent d'agglutination, lesdites particules comportant éventuellement l'une quelconque ou une combinaison de particules polymères, de particules d'or, de particules d'argent, de nanoparticules et de microparticules.

9. Procédé selon l'une quelconque des revendications 1 à 8, destiné à être utilisé pour l'agglutination d'une suspension de particules polymères ou destiné à être utilisé pour l'agglutination d'une suspension de globules rouges.

10. Procédé selon l'une quelconque des revendications 1 à 8, dans lequel ledit fluide est du sang comprenant au moins des globules rouges et, éventuellement, ledit agent d'agglutination est un agent d'agglutination chimique utilisé pour agglutiner les globules rouges pour la détermination du taux d'hématocrite.

11. Système comprenant :
un dispositif DMF d'électromouillage à deux plaques comprenant :
une première plaque, une seconde plaque espacée de ladite première plaque, l'une parmi lesdites première et seconde plaques comportant une pluralité d'électrodes motrices ; et
une surface sur l'une quelconque parmi la première plaque ou la seconde plaque comportant un tensioactif sous une forme pré-séchée recouvrant la surface à des emplacements présélectionnés, une autre surface sur l'une quelconque parmi la première plaque ou la seconde plaque comportant un agent d'agglutination sous une forme pré-séchée recouvrant l'autre surface à des emplacements présélectionnés ;
une caméra ; et
des moyens de traitement d'images configurés pour effectuer le traitement d'images tel que défini dans l'une quelconque des revendications 1 à 10.

12. Système selon la revendication 11, comprenant en outre un microprocesseur connecté à une alimentation électrique et à ladite pluralité d'électrodes motrices et programmé avec des instructions pour fournir une alimentation électrique auxdites électrodes motrices selon un modèle présélectionné pour déplacer des gouttelettes d'échantillon de fluide étudiées pour la présence d'analytes présélectionnés situés en leur sein et un agent d'agglutination sur les électrodes.

13. Système selon la revendication 11 ou 12, dans lequel la surface revêtue par le tensioactif et la surface revêtue par l'agent d'agglutination sont soit différentes, soit identiques.
